# EUROPEAN PATENT APPLICATION

(11) **EP 4 272 575 A1**
(43) Date of publication of application: **08.11.2023**
(21) Application number: 21915814.4
(22) Date of filing: 29.12.2021
(51) Int. Cl.: A23L 13/20, A23L 13/40, A61K 35/32, A61P 37/00

(54) **IMMUNO-POTENTIATING ENZYMATIC EXTRACT OF DEER ANTLER THAT EXCELLENTLY ENHANCES ACTIVITY OF IMMUNE CELL INCLUDING NK CELL**

(30) Priority: 30.12.2020 KR 20200187513; 28.12.2021 KR 20210189206
(71) Applicant: Yuhan Care Co., Ltd., Seoul 07335 (KR)
(72) Inventor: KANG, Jongsoo, Seoul 01370 (KR); CHUNG, Kyung In, Seoul 05096 (KR); PARK, Il Bum, Suwon-si Gyeonggi-do 16325 (KR); PARK, Hyun Je, Gunpo-si Gyeonggi-do 15889 (KR); SONG, Aeri, Hwaseong-si Gyeonggi-do 18441 (KR); EOM, Semi, Seoul 07283 (KR); JEON, Hye Jin, Yongin-si Gyeonggi-do 16995 (KR); BAEK, Sin Hwa, Cheonan-si Chungcheongnam-do 31145 (KR)
(74) Representative: V.O.
(86) International application number: PCT/KR2021/020127
(87) International publication number: WO 2022/146016

(57) **Abstract**

The present disclosure relates to an immuno-potentiating composition, more specifically to a composition containing an enzymatic extract of deer antler with excellent NK cell activity as an active ingredient, which exhibits immuno-potentiating effect by proliferating immune cells and particularly enhancing NK cell activity and, as such, can be used as a food composition and further as a health functional food or pharmaceutical composition for immuno-potentiation.

## Description

### [Technical Field]

The present disclosure relates to an immuno-potentiating composition, more specifically to an immuno-potentiating food composition, particularly an immuno-potentiating health functional food or pharmaceutical composition, containing an enzymatic extract of deer antler with superior NK cell activity as an active ingredient.

### [Background Art]

With the improved standard of living and westernized dietary habits, the incidence of lifestyle-related diseases (adult diseases) such as obesity, diabetes, hypertension, heart disease, etc. is increasing due to overnutrition, lack of exercise, etc. In addition, with accelerated transition to an aging society because of extended life expectancy, interests in health promotion are increasing.

Health promotion refers to aiming at increasing resistance to diseases, especially chronic degenerative diseases or infections, and continued stresses and enhancing daily activities. As a measure, it is necessary to manage the whole lifestyle such as improvement of nutrition, enhancement of immune function, proper exercise and rest, continuous mental activities, etc.

If immunity is high, a disease does not arise well usually and, even if a disease has arisen, it is recovered quickly. It is obvious that life expectancy is extended if such a healthy state is maintained continuously. Therefore, it can be said that immunity is directly related to human health and life span and it is essential to manage immunity in order to maintain health and extend and life span.

Immunity refers to a mechanism for physiologically recognizing foreign and endogenous substances in the body of human and animals and maintaining homeostasis. The foreign substance is called an antigen, and numerous types of white blood cells and proteins are involved in the process of removing it. Immunity is largely divided into innate immunity possessed from birth and acquired immunity which is acquired after birth through adaptation to lives, etc.

Innate immunity is also called natural immunity or natural resistance. It responds nonspecifically to antigens and has no special memory function. The innate immune systems include skin and mucous tissues that block the invasion of antigens, strongly acidic gastric acid, the complement system in blood, etc. Cells involved in innate immunity include macrophages responsible for phagocytosis, polymorphonuclear leukocytes, NK cells capable of killing infected cells, etc. In addition, there are cytokines produced by macrophages. In fact, most of infections are defended by the innate immunity.

Infection is recognized first mainly by macrophages. In the process of phagocytosis of antigens by macrophages, cytokines that mediate immune responses are produced.

The cytokines are glycoproteins used as signaling molecules that regulate and stimulate the body's defense system, and play important functions in immunity, infectious diseases, hematopoietic function, tissue recovery, and cellular development and growth. Since cytokines have the function of gathering immune cells in an infected area by inducing inflammation at the site, they are also called inflammatory cytokines. The most important immune function is performed by a group of cytokines known as interleukins, interferon gamma and tumor necrosis factors. Among these, the interleukins are substances that play an important role in the body's defense by regulating immune response as being involved in various stages of the immune response, and include IL-1, IL-6, IL-12, etc.

And, the NK (natural killer) cells perform the function of removing host cells infected by tumor cells, bacteria, intracellular parasites or viruses without prior sensitization by antigens, rejecting inappropriate bone marrow transplantation, regulating immune response of T cells, etc. The NK cells act in the front line of the immune system's defense mechanism in vivo. It is known that the ability of attacking and removing target cells is increased when the activity of the NK cells is excellent.

Deer antler is a product made by cutting and drying the young antler of male sika deer, elk or red deer with thick hair, which has not been ossified yet or is slightly ossified. It refers to the antler of male deer within 2 months after the start of growth, whose tissues are soft and evenly covered with hairs because keratinization has not occurred yet. Whereas the deer antler which has not keratinized is slightly soft, it becomes hard in the fall due to keratinization and the efficacy as the deer antler is decreased. It is called deer horn.

According to ancient literature such as Dongui Bogam, etc., deer antler is known to have many pharmacological efficacies such as tonification, promotion of growth and development, blood tonification, hematopoiesis, excitement of internal organs, treatment of heart failure, enhancement of physical functions, enhancement of physical vitality, enhancement of diuretic functions of kidneys, etc. As efficacies in animals, promotion of growth, hematopoiesis, lowering of serum cholesterol, promotion of protein synthesis, prevention of aging, enhancement of activity on spinal nerves, alleviation of pain, relief of fatigue, enhancement of immune activity, sedation, etc. in whit rats have been reported

As the physiologically active ingredients of deer antler, amino acids, calcium phosphate, calcium carbonate, collagen, phospholipids, chondroitin, glucosamine, pantocrine, gangliosides, hyaluronic acid, etc. are known.

Conventional methods of taking deer antler in the oriental medicine include mixing of dried deer antler with various herbal medicines, followed by pulverization, hot extraction using water as a solvent and filtration, and mixing of dried deer antler with various herbal medicines, followed by pulverization into a powder and preparation into a pill. The intake of the extract is advantageous in that it is easy to absorb the active ingredients of the deer antler, but there is limitation in that the active ingredients contained in the extract cannot be utilized. The pill is advantageous in that the deer antler itself can be ingested, but there is a problem that the active ingredients cannot be absorbed completely in the body.

Korea Patent Publication No. 2005-0090041 describes a method of pulverizing and extracting deer antler and then treating the same with a protease and hydrochloric acid. However, the process is complicated because extraction, protease treatment and hydrochloric acid treatment are performed sequentially and there is a problem that physiologically active substances useful for the human body may be degraded.

And, Korea Patent Publication No. 2013-0078724 describes a method of treating deer antler with pepsin and a lipolytic enzyme under an acidic condition and then treating with a protease under a neutral condition. However, it merely describes that the contents of free amino acids and phospholipids have been increased and does not describe actual physiological activities or specific functions.

### [References of Related Art]

### [Patent Documents]

(Patent document 1) Korea Patent Publication No. 2005-0090041.
(Patent document 2) Korea Patent Publication No. 2013-0078724.

### [Disclosure]

### [Technical Problem]

The present disclosure is directed to providing an enzymatic extract of deer antler with superior NK cell activity, obtained by degrading deer antler with an enzyme of IUBMB EC 3.4.11.1 or an enzyme of IUBMB EC 3.4.24.28, as an active ingredient and an immuno-potentiating food composition containing the same.

The present disclosure is also directed to providing a method for preparing an enzymatic extract of deer antler having immuno-potentiating activity, which includes: a freeze-dried deer antler preparation step of freeze-drying raw deer antler or frozen deer antler; and an enzyme treatment step of adding 50-500 parts by weight of water and 0.01-1 part by weight of an enzyme to 10 parts by weight of the freeze-dried deer antler and conducting reaction at 30-60 °C for 1-48 hours, wherein the enzyme is an enzyme of IUBMB EC 3.4.11.1 or an enzyme of IUBMB EC 3.4.24.28.

The present disclosure is also directed to providing an immuno-potentiating pharmaceutical composition containing an enzymatic extract of deer antler with superior NK cell activity obtained by degrading deer antler with an enzyme of IUBMB EC 3.4.11.1 or an enzyme of IUBMB EC 3.4.24.28 as an active ingredient.

### [Technical Solution]

The present disclosure provides an immuno-potentiating food composition containing an enzymatic extract of deer antler obtained by degrading deer antler with an enzyme of IUBMB EC 3.4.11.1 or an enzyme of IUBMB EC 3.4.24.28 as an active ingredient.

In an exemplary embodiment of the present disclosure, the enzymatic extract of deer antler may be an enzymatic extract of deer antler obtained by degrading deer antler with an enzyme of IUBMB EC 3.4.11.1 or an enzyme of IUBMB EC 3.4.24.28.

In an exemplary embodiment of the present disclosure, the enzyme of IUBMB EC 3.4.11.1 may be a complex of a protease and a peptidase derived from a mold.

In an exemplary embodiment of the present disclosure, the complex of a protease and a peptidase derived from a mold may be a complex of an endoprotease and an exopeptidase derived from *Aspergillus oryzae.*

In an exemplary embodiment of the present disclosure, the complex of an endoprotease and an exopeptidase may be Flavourzyme.

In an exemplary embodiment of the present disclosure, the enzyme of IUBMB EC 3.4.24.28 may be a neutral protease derived from *Bacillus.*

In an exemplary embodiment of the present disclosure, the neutral protease derived from *Bacillus* may be a neutral Zn metalloendoprotease produced by submerged fermentation of *Bacillus amyloliquefaciens.*

In an exemplary embodiment of the present disclosure, the neutral Zn metalloendoprotease may be Neutrase.

In an exemplary embodiment of the present disclosure, the enzymatic extract of deer antler may be an enzymatic extract of deer antler obtained by hydrolyzing dried antler obtained by freeze-drying raw deer antler or frozen deer antler by treating with an enzyme.

The present disclosure also provides a method for preparing an enzymatic extract of deer antler having immuno-potentiating activity, which includes: a freeze-dried deer antler preparation step of freeze-drying raw deer antler or frozen deer antler; and an enzyme treatment step of adding 50-500 parts by weight of water and 0.01-1 part by weight of an enzyme to 10 parts by weight of the freeze-dried deer antler and conducting reaction at 30-60 °C for 1-48 hours, wherein the enzyme is an enzyme of IUBMB EC 3.4.11.1 or an enzyme of IUBMB EC 3.4.24.28.

The present disclosure also provides an immuno-potentiating pharmaceutical composition containing an enzymatic extract of deer antler obtained by degrading deer antler with an enzyme of IUBMB EC 3.4.11.1 or an enzyme of IUBMB EC 3.4.24.28 as an active ingredient.

In an exemplary embodiment of the present disclosure, the enzymatic extract of deer antler may be an enzymatic extract of deer antler obtained by degrading deer antler with an enzyme of IUBMB EC 3.4.11.1 or an enzyme of IUBMB EC 3.4.24.28.

### [Advantageous Effects]

A composition containing an enzymatic extract of deer antler with superior NK cell activity of the present disclosure as an active ingredient, which proliferates immune cells or activates the function of immune cells, can be used as a food composition and further as a health functional food or pharmaceutical composition for immuno-potentiation.

### [Brief Description of Drawings]

FIG. 1 shows a result of investigating the effect of enhancing antigen-stimulated leukocytic immunoactivity of an enzymatic extract of deer antler in Test Example 2 by analyzing the expression of immune-related cytokines using NanoString. Red color indicates up-regulation and blue color indicates down-regulation.
FIG. 2 shows a result of comparing the production of nitric oxide by macrophages depending on the type and concentration of enzymatic extracts of deer antler in Test Example 3.
FIG. 3 shows a result of comparing the expression level of the iNOS gene depending on the type and concentration of enzymatic extracts of deer antler in Test Example 4.
FIG. 4 shows a result of comparing the expression level of the COX2 gene depending on the type and concentration of enzymatic extracts of deer antler in Test Example 4.
FIG. 5 shows a result of comparing the relative expression level of TNF-α depending on the type and concentration of enzymatic extracts of deer antler in Test Example 5.
FIG. 6 shows a result of comparing the relative expression level of IFN-γ depending on the type and concentration of enzymatic extracts of deer antler in Test Example 5.
FIG. 7 shows a result of comparing the relative expression level of IL-6 depending on the type and concentration of enzymatic extracts of deer antler in Test Example 5.
FIG. 8 shows a result of comparing the relative expression level of IL-1β depending on the type and concentration of enzymatic extracts of deer antler in Test Example 5.
FIG. 9 shows a result of comparing the proliferation of macrophages depending on the type and concentration of enzymatic extracts of deer antler and culturing time in Test Example 6.
FIG. 10 shows a result of comparing the NK cell activity depending on the type and concentration of enzymatic extracts of deer antler in an immunosuppression animal model in Test Example 7.
FIG. 11 shows a result of comparing the concentration of TNF-α depending on the type and concentration of enzymatic extracts of deer antler in an immunosuppression animal model in Test Example 7.
FIG. 12 shows a result of comparing the concentration of interferon gamma (IFN-γ) depending on the type and concentration of enzymatic extracts of deer antler in an immunosuppression animal model in Test Example 7.

### [Best Mode]

Hereinafter, the present disclosure is described in detail.

The inventors of the present disclosure have identified that an enzymatic extract of deer antler, particularly an enzymatic extract of deer antler obtained by degrading deer antler with an enzyme of IUBMB EC 3.4.11.1 or an enzyme of IUBMB EC 3.4.24.28, specifically an enzymatic extract of deer antler obtained by degrading deer antler with an enzyme of IUBMB EC 3.4.11.1, has superior NK cell activity and exhibits immuno-potentiating activity by enhancing the expression of immune-related cytokines.

The deer antler refers to the non-ossified antler of male deer within 2 months after birth.

The deer antler may be dried deer antler obtained by natural drying over several weeks under low-humidity environment. Specifically, dried antler obtained by freeze-drying raw deer antler or frozen deer antler may be used in the present disclosure.

When raw deer antler or frozen deer antler is treated with hot water for sterilization before natural drying or freeze-drying, nonspecific and nonuniforrm degradation by endogenous enzymes may occur before the temperature of the core part of the deer antler reaches the deactivation temperature of the enzymes. Accordingly, an enzymatic extract of deer antler with uniform quality and excellent immuno-potentiating activity can be prepared by freeze-drying raw deer antler or frozen deer antler without treating with hot water. The freeze-dried deer antler may be used after sterilization by a common method, e.g., by treating at 120-130 °C for 30 seconds to 20 minutes.

An enzymatic extract of deer antler prepared using an enzyme of IUBMB EC 3.4.23.1, e.g., pepsin or aspartic proteinase, does not exhibit immuno-potentiating effect. Pepsin is an aspartic endopeptidase and selectively cleaves a hydrophobic, particularly aromatic, amino acid residue.

An enzymatic extract of deer antler prepared using the enzyme of IUBMB EC 3.4.11.1, e.g., A-LAP, acidic M17 leucine aminopeptidase, AcLAP, adipocyte-derived leucine aminopeptidase, aminopeptidase, "Flavourzyme" (Novozyme), etc., exhibits remarkably superior immuno-potentiating effect as compared to the conventional deer antler hot water extract or supercritical deer antler extract. The Flavourzyme is a complex of an endoprotease and an exopeptidase produced from submerged fermentation of *Aspergillus oryzae.*

The enzyme of IUBMB EC 3.4.24.28 is a neutral protease derived from *Bacillus,* e.g., bacillolysin, *Bacillus* metalloendopeptidase, Anilozyme P10; *Bacillus* metalloproteinase, megateriopeptidase, "Neutrase" (Novozyme), etc. An enzymatic extract of deer antler prepared using the enzyme of IUBMB EC 3.4.24.28 exhibits remarkably superior immuno-potentiating effect as compared to the conventional deer antler hot water extract or supercritical deer antler extract. The Neutrase is a neutral Zn metalloendoprotease produced from submerged fermentation of *Bacillus amyloliquefaciens.* In addition, an enzymatic extract of deer antler treated by a neutral metalloproteinase derived from the same bacteria, such as "Protease N 'Amano‴ (Amano Enzyme), "Corolase N" (AB Enzymes), etc., also exhibits remarkably superior immuno-potentiating effect as compared to the conventional deer antler hot water extract or supercritical deer antler extract.

In contrast, an enzymatic extract of deer antler prepared by treating with an enzyme of IUBMB EC 3.4.21.62, e.g., Alcalase, subtilisin, colorase N, ALK-enzyme; bacillopeptidase A; bacillopeptidase B; *Bacillus subtilis* alkaline proteinase bioprase; bioprase AL 15; bioprase APL 30; colistinase; subtilisin J; subtilisin S41; subtilisin Sendai; subtilisin GX; subtilisin E; subtilisin BL; subtilisin A (type VIII); genenase I; Esperase; maxatase; thermoase PC 10; protease XXVII; thermoase; superase; subtilisin DY; subtilopeptidase; SP 266; Savinase 8.0L; Savinase 4.0T; kazusase; protease VIII; Optimase; Opticlean; *Bacillus subtilis* alkaline proteinase; protein A 3L; Savinase; Savinase 16.0L; Savinase 32.0 L EX; orientase 10B; protease S and similar serine endopeptidases does not exhibit immuno-potentiating effect.

In addition, an enzymatic extract of deer antler prepared by treating with "Protamex" (Novozyme), which is a complex of an enzyme of IUBMB EC 3.4.24.28 and an enzyme of IUBMB EC 3.4.21.62, does not exhibit immuno-potentiating effect.

The enzymatic extract of deer antler is prepared by adding a proper amount of deer antler and an enzyme to water. For example, 50-500 parts by weight, specifically 100-400 parts by weight, of water and 0.01-1 part by weight, specifically 0.02-0.8 parts by weight, more specifically 0.05-0.5 parts by weight, of an enzyme may be added for 10 parts by weight of dried deer antler such as freeze-dried deer antler, but the addition amounts may be changed as desired.

The enzyme treatment may be performed within an appropriate temperature range, specifically at 30-60 °C, more specifically at 40-55 °C.

The enzyme treatment may be performed for an appropriate time depending on the amount of a peptide produced by the enzyme treatment, the molecular weight of the peptide and its profile. It may be performed specifically for 1 hour or longer, more specifically for 2 hours or longer, further more specifically for 3 hours or longer. Even if the enzyme treatment is performed longer than a specific time, the effect on production yield is almost negligible and only the process cost may increase. Therefore, the enzyme treatment is performed specifically for 48 hours or shorter, more specifically for 24 hours or shorter, further more specifically for 12 hours or shorter.

A step of deactivating the enzyme may be further included after the enzyme treatment step. The deactivation of the enzyme may be achieved by any method known to those having ordinary skill. For example, the deactivation of the enzyme may be achieved by treating at 85 °C or higher for 20 minutes or longer.

The 'enzymatic extract of deer antler' includes a reaction product obtained through the enzyme treatment of deer antler, a fraction obtained therefrom, a hydrolysate or concentrate of the fraction and a purification product obtained by purifying or separating the same, and also includes a dried product of the reaction product, fraction, concentrate or purification product and a powder obtained by pulverizing the same.

The concentrate or purification product may be prepared by separation through centrifugation, filtration through an ultrafiltration membrane having a molecular weight cutoff value, or separation by various chromatographies (designed for separation based on size, charge, hydrophobicity or affinity).

The present disclosure relates to an immuno-potentiating food composition containing an enzymatic extract of deer antler as an active ingredient.

The 'food composition' contains, in addition to the enzymatic extract of deer antler as an active ingredient, food ingredients commonly used for preparation of food and described in the Standards and Specifications of Food ('Food Code') and food additives described in the Food Additives Code.

They include, although not being specially limited thereto, for example, proteins, carbohydrates, fats, nutrients, seasonings and flavorants. The carbohydrate may be a monosaccharide, e.g., glucose, fructose, etc.; a disaccharide, e.g., maltose, sucrose, lactose, etc.; an oligosaccharide or polysaccharide, e.g., dextrin, starch syrup, cyclodextrin, etc.; a sugar alcohol, e.g., xylitol, sorbitol, erythritol, etc. The flavorant may be a natural flavorant [thaumatin or stevia extract (e.g., rebaudioside A, glycyrrhizin, etc.]) or a synthetic flavorant (saccharin, aspartame, etc.).

When a food composition containing the enzymatic extract of deer antler as an active ingredient is prepared, the content of the enzymatic extract of deer antler is not specially limited as long as it can exhibit immuno-potentiating effect. For example, it may be contained at a content of 0.1-99 wt%, 0.5-95 wt%, 1-90 wt%, 2-80 wt%, 3-70 wt%, 4-60 wt% or 5-50 wt%.

The amount of the enzymatic extract of deer antler in the food composition may be selected adequately by those having ordinary skill although it varies depending on the condition and body weight of a user, presence or absence of a disease and period. For example, based on a daily dose, the amount may be 1-5,000 mg, specifically 5-2,000 mg, more specifically 10-1,000 mg, further more specifically 20-800 mg, most specifically 50-500 mg. The number of administration is not specially limited but may be adjusted by those having ordinary skill within a range of 3 times a day to once a week. In the case of long-term intake for the purpose of health or hygiene, the amount may be less than the above-described ranges.

For example, the food composition may be in the form of a powder, a granule, a tablet, a capsule, a pill, an extract, a jelly, a tea bag or a beverage, although not being specially limited thereto.

In addition, the enzymatic extract of deer antler may be added to general foods to impart immuno-potentiating function. The foods to which it can be added are not specially limited but may include, for example, the confectionery, bread or rice cakes, processed cocoa products or chocolates, processed meat or egg products, processed fish meat products, tofu or jelly, noodles, teas, coffee, beverages, special-purpose foods, fermented soy products, seasoned foods, dressings, kimchi, salted seafood, pickled foods, stewed foods, alcoholic beverages, dried meat and other foods exemplified in the Standards and Specifications of Food ('Food Code') according to Article 7 of the Food Sanitation Act. In addition, it can be added to the dairy products, processed meat products, packaged meat and processed egg products exemplified in the Processing Standards and Ingredient Specifications of Livestock Products ('Livestock Products Code') according to Article 4 of the Livestock Products Sanitary Control Act.

Since the enzymatic extract of deer antler has superior NK cell activity and enhances the expression of immune-related cytokines, a food composition containing the enzymatic extract of deer antler as an active ingredient may be used as a "health functional food that helps immune-potentiation" on its own.

The 'health functional food' refers to a food prepared (including processing) according to legal standards using raw materials or ingredients with useful functions for the human body (Article 3(1) of the Health Functional Foods Act). The 'health functional food' may have differences in terminology or scope from country to country, but may encompass the 'dietary supplement' of the USA, the 'food supplement' of Europe, the 'health functional food' or 'food for special health use (FoSHU)' of Japan, the 'health food' of China, etc.

The food composition or health functional food may further contain a food additive, and the suitability as the food additive is determined according to the standards and specifications in accordance with the general rules and general test methods of the 'Food Additives Code' unless specified otherwise.

In addition, the health functional food may further contain, together with the enzymatic extract of deer antler, a health functional food ingredient related with immune-potentiation recognized as a 'functional ingredient' used in a "health functional food that helps immune-potentiation", such as L-glutamine, germanium yeast, *Phellinus linteus,* heat-treated dry *Enterococcus faecalis* powder, angelica extract, *Cordyceps militaris* ethanol extract, spirulina, chlorella, purified enteric bacillus culture of cheonggukjang (poly-γ-glutamate potassium), *Lentinus edoes* mycelium, yeast beta-glucan, ginseng polysaccharide extract, etc.

The present disclosure relates to an immuno-potentiating pharmaceutical composition containing the enzymatic extract of deer antler as an active ingredient.

The 'pharmaceutical composition' may further contain, in addition to the enzymatic extract of deer antler which has superior NK cell activity and enhances the expression of immune-related cytokines as an active ingredient, a suitable carrier, excipient and diluent commonly used for preparation of a pharmaceutical composition, etc.

The 'immuno-potentiating pharmaceutical composition' may be an immuno-anticancer agent or an anticancer adjuvant. The immuno-anticancer agent kills cancer cells by inducing the expression of immune factors and thereby increasing the activity of immune cells. The cancer cells may be the cancer cells of a solid cancer. More specifically, the solid cancer may be selected from a group consisting of colorectal cancer, breast cancer, lung cancer, stomach cancer, epithelial ovarian cancer, brain cancer, skin cancer and liver cancer, although not being limited thereto. The anticancer adjuvant may alleviate side effects caused by the administration of an anticancer agent, and the side effects caused by the administration of the anticancer agent may be selected from a group consisting of decrease in body weight or immune factors and cachexia.

The 'immuno-potentiating pharmaceutical composition' may be an immuno-potentiating agent that enhances the efficacy of an antiviral vaccine. The immuno-potentiating agent may be administered concurrently or sequentially with the antiviral vaccine.

The antiviral vaccine may be a human vaccine for prevention of influenza, hepatitis A, hepatitis B, hepatitis C, herpes simplex virus (type 2), poliomyelitis, diphtheria, pertussis, *Haemophilus influenzae* B (Hib), measles, mumps, rubella, typhoid fever, chicken pox, dengue fever, Epstein-Barr virus infection, human papilloma virus infection, pneumococcal infection, meningococcal infection, viral meningitis, rotavirus infection, tick-borne encephalitis, traveler's diarrhea, cholera, yellow fever or tuberculosis.

The 'carrier' is a compound that facilitates the delivery of a compound into a cell or a tissue. The 'diluent' is a compound that is diluted in water, which not only stabilizes the biologically active form of a target compound but also dissolves the compound.

As the carrier, excipient or diluent, lactose, glucose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, etc. may be used, although not being specially limited thereto.

The dosage of the pharmaceutical composition may vary depending on the age, sex and body weight of a patient and, above all, the condition of the subject to be treated, the specific category or type of a disease to be treated, administration route, and the properties of the therapeutic agent used.

The dosage of the pharmaceutical composition is determined adequately depending on the degree of absorption of the active ingredient in the body, the rate of excretion, the age, body weight, sex and condition of a patient or an animal to be treated, the severity of a disease to be treated, etc. It is preferred to administer usually 0.1-1,000 mg/kg, specifically 1-500 mg/kg, more specifically 5-250 mg/kg, most specifically 10-100 mg/kg per day. A unit-dosage formulation formulated in this way may be administered several times at regular intervals as needed.

The pharmaceutical composition may be administered individually as a prophylactic or therapeutic agent or in combination with other therapeutic agents either sequentially or simultaneously.

The pharmaceutical composition may be formulated into an oral formulation such as a powder, a granule, a tablet, a capsule, a troche, a suspension, an emulsion, a syrup, an aerosol, etc. or a parenteral formulation such as a sterilized aqueous solution, a nonaqueous solution, a suspension, an emulsion, a lyophilized formulation, a suppository, etc. according to common methods.

For the formulation, a commonly used diluent or excipient such as a filler, an extender, a binder, a wetting agent, a disintegrant, a surfactant, etc. may be used.

Solid formulations for oral administration include a tablet, a pill, a powder, a granule, a troche, etc., and the solid formulation may be prepared by mixing the enzymatic extract of deer antler with at least one excipient, e.g., starch, calcium carbonate, sucrose, lactose, gelatin, etc. In addition to a simple excipient, a lubricant such as magnesium stearate or talc may also be used. Liquid formulations for oral administration include a suspension, a liquid for internal use, an emulsion, a syrup, etc., and may contain, in addition to a commonly used simple diluent such as water or liquid paraffin, various excipients such as a wetting agent, a sweetener, an aromatic, a preservative, etc.

As a solvent for a nonaqueous solution or a suspension for formulations for parenteral administration, propylene glycol, polyethylene glycol, vegetable oil such as olive oil, an injectable ester such as ethyl oleate, etc. may be used. As a base for a suppository, witepsol, macrogol, Tween 61, cocoa butter, laurin butter, glycerogelatin, etc. may be used.

Hereinafter, the present disclosure is described in more detail through specific examples. However, these examples are only for describing the present disclosure more specifically, and it will be obvious to those having ordinary knowledge in the art that the scope of the present disclosure is not limited by them.

### Preparation Example 1: Preparation of deer antler powder

Blood-containing frozen deer antler (New Zealand) was washed, pulverized into a suitable size, freeze-dried, pulverized again, and then heat-treated at 123 °C for 7 minutes to prepare a freeze-dried deer antler powder.

### Preparation Example 2: Preparation enzymatic extracts of deer antler

10 g of the deer antler powder of Preparation Example 1 was mixed with 199 mL of water. The mixture was stirred at 100 rpm in a water bath at temperatures for different enzymes (e.g., 60 °C for Alcalase, and 50 °C for Flavourzyme, Neutrase and Protamex). After adding 1 mL of each enzyme solution (100 mg/mL) at an enzyme:substrate ratio of 1:100, the mixture was enzyme-treated for 23 hours under stirring while maintaining the temperature. After deactivating the enzyme by maintaining temperature at 85 °C or above in a 95 °C water bath for 20 minutes, followed by cooling, the enzyme degradation product was centrifuged at 25 °C. The supernatant was diluted with 200 mL of water, filtered through Celite 545 in vacuo, sterilized, dried and then prepared into a powder. The powder was stored at -20 °C for analysis of physiological activity, and the samples were named 1F, 2F, 3F and 5F.

For pepsin, 10 g of the deer antler powder of Preparation Example 1 was mixed with 199 mL of dilute hydrochloric acid at pH 2. The mixture was stirred at 100 rpm in a water bath at 50 °C. After adding 1 mL of a pepsin solution (100 mg/mL) at an enzyme:substrate ratio of 1:100, the mixture was enzyme-treated for 23 hours under stirring while maintaining the temperature. After stopping degradation by adding 8 mL of 6 M sodium hydroxide to adjust pH to 5.5-6 and deactivating the enzyme by maintaining temperature at 85 °C or above in a 95 °C water bath for 20 minutes, followed by cooling, the enzyme degradation product was centrifuged at 25 °C. The supernatant was diluted with 200 mL of water, filtered through Celite 545 in vacuo, sterilized, dried and then prepared into a powder. The powder was stored at -20 °C for analysis of physiological activity, and the sample was named 4F.

### Preparation Example 3: Preparation control groups

As control groups of the enzymatic extract of deer antler, a deer antler supercritical extract, a deer antler hot water extract and VARNZ RepaiRx were named C1, C2 and C3, respectively.

The deer antler supercritical extract (C1) was prepared by extracting the deer antler powder of Preparation Example 1 using carbon dioxide and ethanol as solvents under the condition of extraction pressure 300 bar, extraction temperature 40 °C, separation pressure 54 bar, separation temperature 40 °C, supply weight 49.7 g, average carbon dioxide flow rate 7.9 g/min, average ethanol flow rate 2.0 g/min, carbon dioxide supply ratio 22:1 and ethanol supply ratio 3:1, while recovering the sample at 15-minute intervals and drying the same under reduced pressure.

The deer antler hot water extract (C2) was prepared by adding a mixture of 10 g of the deer antler powder of Preparation Example 1 and 200 mL of water in a roundbottom flask, conducting hot water extraction for 3 hours in boiling water, cooling with ice and centrifuging at 25 °C and 25,000 g for 30 minutes. The supernatant was diluted with 200 mL of water, filtered through Celite 545 in vacuo and then dried. The obtained powder was stored at -20 °C for analysis of physiological activity.

VARNZ RepaiRx (C3) was prepared according to US Patent No. 07547761 and then diluted to 1/25.

### Test Example 1: Yield of enzymatic extracts of deer antler

After measuring the concentration of the enzymatic extracts of deer antler 1F-5F prepared in Preparation Example 2 and the control group C2, yield was calculated therefrom. The result is shown in Table 1.

**[Table 1]**

| Samples | Yield (%) |
|---|---|
| 1F | 12.7 |
| 2F | 29.1 |
| 3F | 36.3 |
| 4F | 18.3 |
| 5F | 44.0 |
| C2 | 8.5 |

All the enzymatic extracts of deer antler 1F-5F showed higher yield than the deer antler hot water extract (C2). Among the enzymatic extracts of deer antler, the enzymatic extract of deer antler using Flavourzyme (2F), the enzymatic extract of deer antler using Neutrase (3F) and the enzymatic extract of deer antler using Protamex (5F) showed about 2-3 times higher yield not only as compared to the deer antler hot water extract (C2) but also as compared to the enzymatic extracts of deer antler treated with Alcalase (1F) and pepsin (4F).

### Test Example 2: Effect of enhancing antigen-stimulated leukocytic immunoactivity of enzymatic extracts of deer antler

Leukocytes were separated from the blood samples of 12 volunteers acquired from the NZ Blood Service (Wellington, New Zealand). A red blood cell lysis buffer (TAC buffer: 85 mM Tris, 78 mM NH₄Cl, Sigma-Aldrich, St. Louis, MO, USA) was used after preheating to 37 °C. After adding 45 mL of the TAC buffer to 5 mL of whole blood, the blood was settled in a water bath at 37 °C for 10 minutes. After centrifuging at 500 x g for 10 minutes and removing the supernatant, the remaining pellets were suspended uniformly in 25 mL of PBS (0.1 M EDTA). After centrifuging in the same manner and removing the supernatant, the remaining pellets were suspended in 5 mL of RPMI 1640 (Sigma-Aldrich, supplemented with 5% FCS). The number of the leukocytes used was 1×10⁷ cells/mL.

After seeding 50 µL of the separated leukocytes onto a 96-well plate and then adding 25 µL of RPMI/FCS (control) or the enzymatic extracts of deer antler of Preparation Example 2 or control groups diluted to 1/100, the cells were incubated in a 5% CO₂ incubator at 37 °C for 3 hours. After adding 25 µL of a stimulator, the cells were incubated in a 5% CO₂ incubator at 37 °C for 2 hours. The stimulator was SEB (200 ng/mL *Staphylococcus* enterotoxin B; Sigma-Aldrich), PWM (2 µg/mL pokeweed mitogen; Sigma-Aldrich) or lipopolysaccharide (LPS) (4 ng/mL LPS; Sigma-Aldrich). After centrifuging at 350 x g and removing the cell lysate from the supernatant, the cell pellets were suspended in 10 µL/well of Buffer RLT.

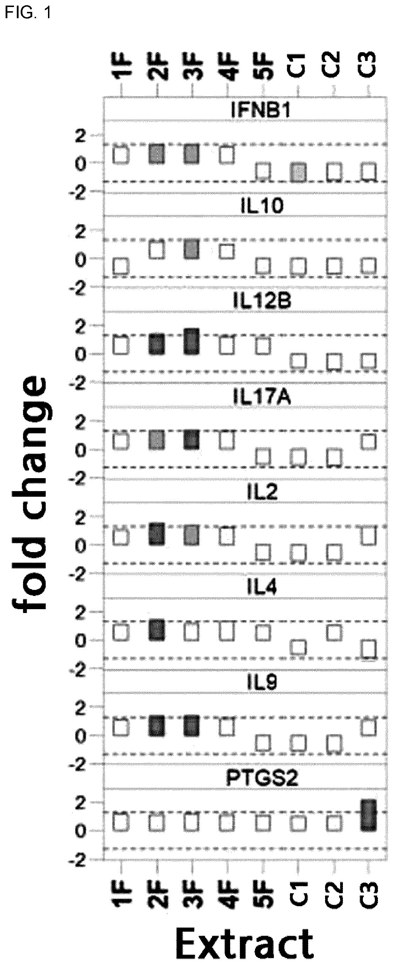

The effect of enhancing antigen-stimulated leukocytic immunoactivity was investigated by analyzing the expression of immune-related cytokines using NanoString. The result is shown in FIG. 1. In FIG. 1, the red color indicates up-regulation and the blue color indicates down-regulation. In FIG. 1, p < 0.05 for the red and blue colors and p < 0.1 for the orange and azure colors.

As a result of investigating the enhancement of the expression of immune-related genes by the enzymatic extracts of deer antler, the enzymatic extract of deer antler using Alcalase (1F) did not increased the expression of immune-related genes regardless of the method of preparing the deer antler powder.

Among the enzymatic extracts of deer antler using Flavourzyme, the freeze-dried enzymatic extract of deer antler (2F) increased the expression of a total of six genes, 1FNB1, 1L12B, IL17A, IL2, IL4 and IL9.

Among the enzymatic extracts of deer antler using Neutrase, the freeze-dried enzymatic extract of deer antler (3F) increased the expression of a total of six genes, IFNB1, IL10, IL12B, IL17A, IL2 and IL9.

Among the enzymatic extracts of deer antler using pepsin, the freeze-dried enzymatic extract of deer antler (4F) did not increase the expression of any gene.

Among the enzymatic extracts of deer antler using Protamex, the freeze-dried enzymatic extract of deer antler (5F) did not increase the expression of any gene.

The control groups C1, C2 and C3 not increase the expression of any immune-related gene, and C3 increased only the expression of the PTGS2 gene.

As a result of investigating the activity of increasing the expression of immune-related cytokines of the enzymatic extracts of deer antler, the enzymatic extract of deer antler using Flavourzyme (2F) and the enzymatic extract of deer antler using Neutrase (3F) showed high activities. When the deer antler was treated with other enzymes or was not treated with any enzyme, the activity of increasing the expression of immune immune-related cytokines was very low.

In the following experiments, the enzymatic extract of deer antler using Flavourzyme (2F), the enzymatic extract of deer antler using Neutrase (3F), the enzymatic extract of deer antler using pepsin (4F), the enzymatic extract of deer antler using Protamex (5F) and the deer antler hot water extract (C2) were used.

### Test Example 3: Effect of enzymatic extracts of deer antler on production of nitric oxide by macrophages

Mouse-derived RAW 264.7 macrophages were cultured in a 5% CO₂ incubator (MCO-20AIC, Sanyo, Japan) at 37 °C using a mixture of 445 mL of DMEM (Cat No. 11995-065, Gibco BRL, U.S.A), 50 mL of FBS (Lot No. AE29155306, Hyclone, USA) and 5 mL of Antibiotic-Antimycotic 100x (Batch No. ADD670076, Biopredic International, France), while subculturing with 2- to 3-day intervals until use.

Before investigating the effect of the enzymatic extracts of deer antler on nitric oxide (NO) production by macrophages, MTT assay was conducted to investigate the cytotoxicity of the samples. The activated RAW 264.7 cells were seeded onto a 96-well plate at 3×10⁴ cells/well and then cultured in a 5% CO₂ incubator at 37 °C for 24 hours. After replacing the medium with the enzymatic extract samples of deer antler prepared to concentrations of 0.001-100 mg/mL, the cells were cultured for 24 hours. Then, after adding 10 µL of CCK-8 (Cat No. CK04-13, Dojindo, Japan) and further culturing the cells in a 5% CO₂ incubator at 37 °C for 1 hour, cell survivability was determined with respect to a normal control group by measuring absorbance at 450 nm using a microplate reader.

The enzymatic extract samples of deer antler 2F, 3F, 5F and C2 did not show cytotoxicity at 0.5, 2.5, 6 and 50 mg/mL or lower.

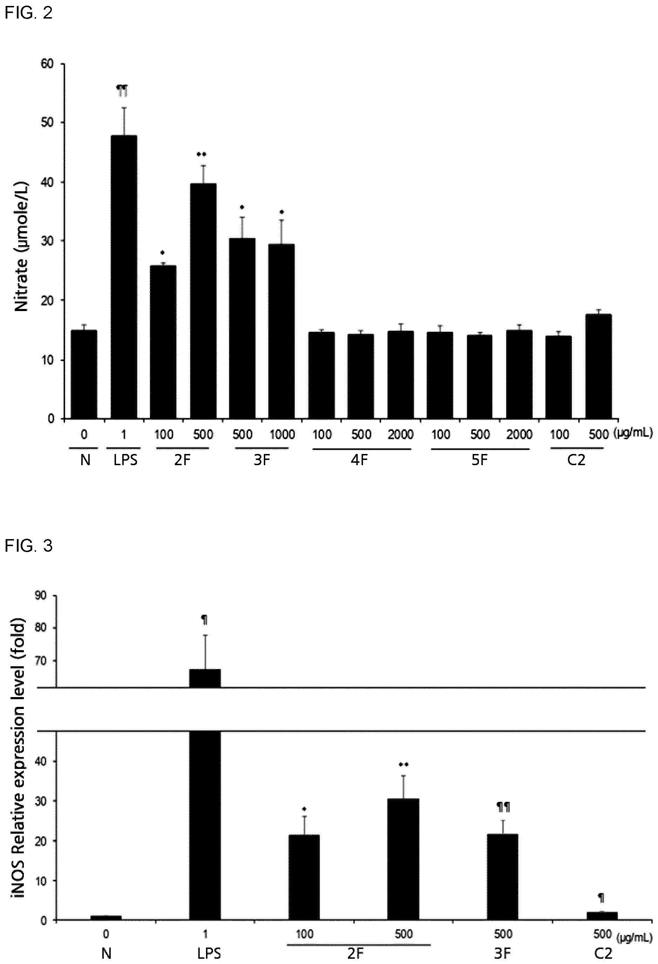

For investigation of the effect on nitric oxide production, RAW 264.7 cells were seeded onto a 6-well plate at 5×10⁵ cells/well and cultured for 24 hours. After removing the culture medium and replacing with a medium containing the positive control group or the samples at different concentrations, the cells were cultured further for 24 hours. The cell culture was recovered and centrifuged at 1000 rpm for 5 minutes. After transferring the supernatant to a fresh tube, NO production was measured using a NO production kit (Cat No. ADI-917-020, Enzo, USA) according to the manufacturer's protocol. The NO production was quantified from the standard curve of absorbance measured at 540 nm using 4-parameter logic regression. The result is shown in FIG. 2.

14.89 µmole/L of nitric oxide was produced for the normal control group (N) not treated with the sample. 47.74 µmole/L of nitric oxide was produced for the positive control group (LPS 1 µg/mL), which was significantly increased as compared to the normal control group (p < 0.01).

The production amount of nitric oxide for the groups treated with the enzymatic extract of deer antler using Flavourzyme (2F) (100, 500 µg/mL) was increased in a concentration-dependent manner with 25.73 and 39.65 µmole/L, respectively, which was significantly increased as compared to the normal control group (p < 0.05 and p < 0.01).

The production amount of nitric oxide for the groups treated with the enzymatic extract of deer antler using Neutrase (3F) (500, 1000 µg/mL) was increased in a concentration-dependent manner with 30.42 and 29.39 µmole/L, respectively, which was significantly increased as compared to the normal control group (p < 0.01 and p < 0.01). However, the increase was about 23.3% lower as compared to the group treated with the enzymatic extract of deer antler using Flavourzyme (2F) at the same concentration (500 µg/mL).

The groups treated with the enzymatic extract of deer antler using Protamex (5F) and the deer antler hot water extract (C2) did not show significant difference from the normal control group.

Nitric oxide production by macrophages is an indicator of the activation of macrophages that provide protection against foreign substances in the mammalian immune system. It was confirmed that the enzymatic extract of deer antler using Flavourzyme (2F) showed the best nitric oxide production, followed by the enzymatic extract of deer antler using Neutrase (3F).

### Test Example 4: Effect of enzymatic extracts of deer antler on increased expression of iNOS and COX2 genes

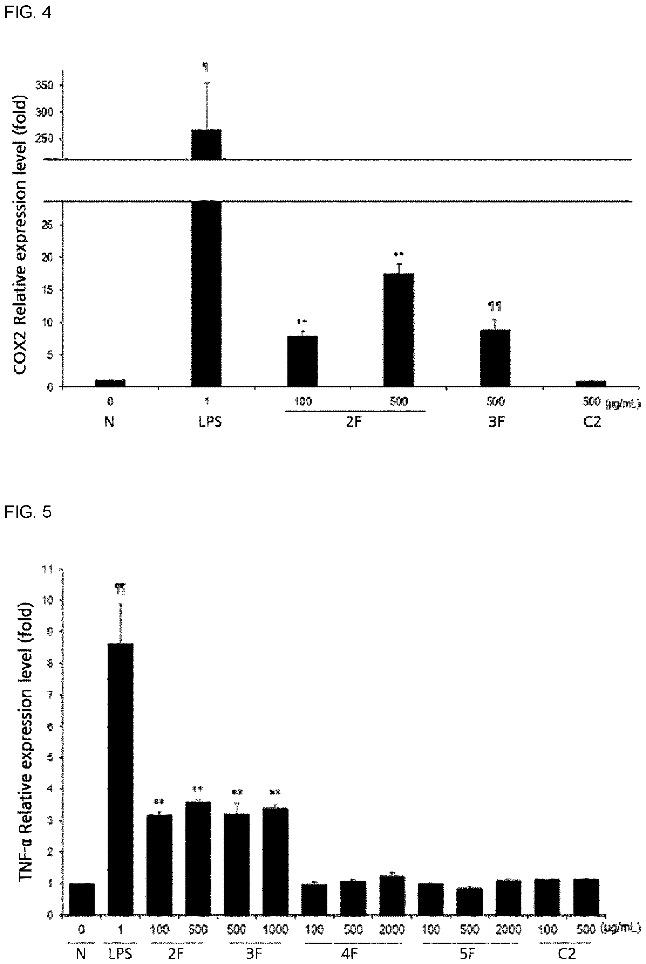

For investigation of the expression level of the iNOS and COX-2 genes, RAW 264.7 cells were seeded onto a 6-well plate at 5×10⁵ cells/well and then cultured for 24 hours. After removing the culture medium and replacing with a medium containing the positive control group or the samples at different concentrations, the cells were cultured further for 24 hours. The cells were collected and total RNAs were extracted using an RNA extract kit (Cat No. 74106, Qiagen, Germany) according to the manufacturer's protocol. The extracted total RNAs were quantified by measuring absorbance at 260 nm and 280 nm using Infinite 200 Pro (Tecan, Switzerland) and then diluted to specified concentrations. Then, the expression level of the iNOS and COX2 genes was measured using a TOPreal One-step kit (Cat No. RT432M, Enzynomics, Korea). All the results were calibrated with β-actin and normalized to the normal control group according to the 2-^{ΔΔ}CT method. The relative expression level of iNOS is shown in FIG. 3, and the relative expression level of COX2 is shown in FIG. 4.

As seen from FIG. 3, the expression level of iNOS in the positive control group (LPS, 1 µg/mL) was increased by 67.37 times, which was statistically significant when compared with the normal control group (p < 0.05).

For the groups treated with the enzymatic extract of deer antler using Flavourzyme (2F) (100, 500 µg/mL), the iNOS expression level was increased significantly as compared to the normal control group as 21.55 and 30.45 times, respectively (p < 0.05 and p < 0.01).

For the group treated with the enzymatic extract of deer antler using Neutrase (3F) (500 µg/mL), the NOS expression level was increased by 21.74 times as compared to the normal control group (p < 0.01), but the increase was about 28.4% lower as compared to the group treated with the enzymatic extract of deer antler using Flavourzyme (2F) of the same concentration (500 µg/mL).

For the group treated with the deer antler hot water extract (C2) (500 µg/mL), the iNOS expression level was increased by 1.93 times as compared to the normal control group, but the increase was much lower when compared with the enzymatic extract of deer antler using Flavourzyme (2F) or the enzymatic extract of deer antler using Neutrase (3F).

As seen from FIG. 4, the expression level of COX2 in the positive control group (LPS, 1 µg/mL) was increased by 264.54 times, which was statistically significant when compared with the normal control group (p < 0.05).

For the groups treated with the enzymatic extract of deer antler using Flavourzyme (2F) (100, 500 µg/mL), the COX2 expression level was increased significantly as compared to the normal control group as 7.82 and 17.49 times, respectively (p < 0.01 and p < 0.01).

For the group treated with the enzymatic extract of deer antler using Neutrase (3F) (500 µg/mL), the COX2 expression level was increased by 8.86 times as compared to the normal control group (p < 0.01), but the increase was about 49.3% lower as compared to the group treated with the enzymatic extract of deer antler using Flavourzyme (2F) of the same concentration (500 µg/mL).

For the group treated with the deer antler hot water extract (C2) (500 µg/mL), the COX2 expression level was not different from the normal control group as 0.98 times.

iNOS is a gene involved in the production of NO through L-arginine in activated macrophages, and COX2 is a gene which is increased by NO production and other cytokines. It was conformed that the expression level of both genes was increased the most remarkably for the enzymatic extract of deer antler using Flavourzyme (2F), followed by the enzymatic extract of deer antler using Neutrase (3F).

### Test Example 5: Effect of enzymatic extracts of deer antler on increase of immune-related cytokines

The relative expression level of the immune-related cytokines TNF-α, IFN-γ, IL-6 and IL-1β was measured using a TOPreal One-step kit in the same manner as in Test Example 4. The results are shown in FIG. 5, FIG. 6, FIG. 7 and FIG. 8, respectively.

As seen from FIG. 5, the expression level of TNF-α in the positive control group (LPS, 1 µg/mL) was increased statistically significantly when compared with the normal control group (N) (p < 0.01).

For the groups treated with the enzymatic extract of deer antler using Flavourzyme (2F) (100, 500 µg/mL), the TNF-α expression level was increased significantly as compared to the normal control group (N) as 3.17 and 3.58 times, respectively (p < 0.01 and p < 0.01).

For the groups treated with the enzymatic extract of deer antler using Neutrase (3F) (500, 1000 µg/mL), the TNF-α expression level was increased significantly as compared to the normal control group (N) as 3.2 and 3.38 times, respectively (p < 0.01 and p < 0.01).

For the groups treated with the enzymatic extract of deer antler using pepsin (4F) (100, 500, 2000 µg/mL), the groups treated with the enzymatic extract of deer antler using Protamex (5F) (100, 500, 2000 µg/mL) and the groups treated with the deer antler hot water extract (C2) (100, 500 µg/mL), there was no difference in the TNF-α expression level as compared to the normal control group at all concentrations.

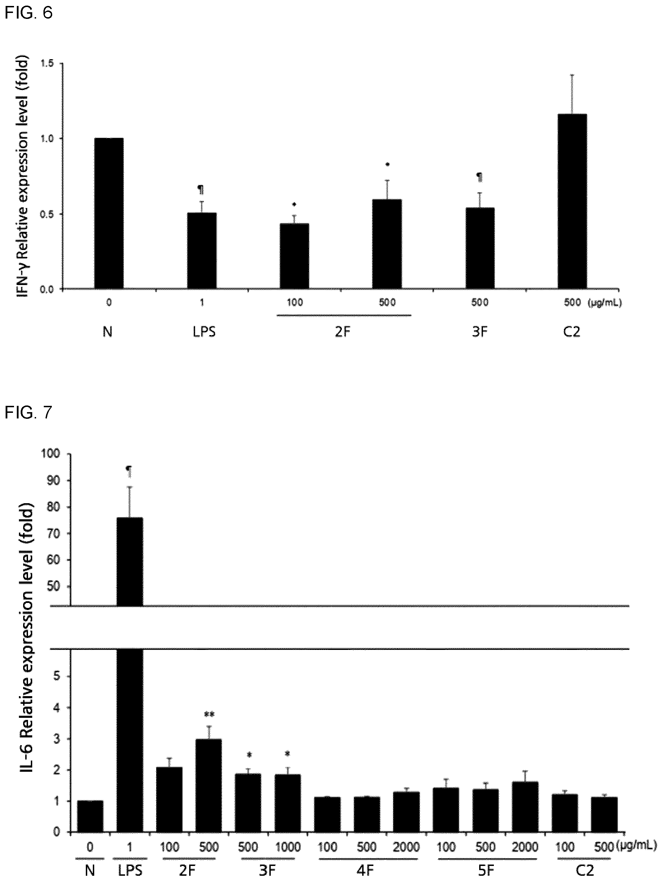

As seen from FIG. 6, the expression level of IFN-γ in the positive control group (LPS 1 µg/mL) was decreased statistically significantly as compared to the normal control group (N) (p < 0.05).

For the groups treated with the enzymatic extract of deer antler using Flavourzyme (2F) (100, 500 µg/mL), the IFN-γ expression level was decreased statistically significantly as compared to the normal control group (N) as 0.44 and 0.59 times, respectively (p < 0.05 and p < 0.05).

For the group treated with the enzymatic extract of deer antler using Neutrase (3F) (500 µg/mL), the IFN-γ expression level was decreased significantly as compared to the normal control group (N) as 0.54 times (p < 0.05).

However, for the group treated with the deer antler hot water extract (C2) (500 µg/mL), there was no difference in the IFN-γ expression level as compared to the normal control group (N).

As seen from FIG. 7, the expression level of IL-6 in the positive control group (LPS 1 µg/mL) was increased statistically significantly as compared to the normal control group (N) (p < 0.05).

For the groups treated with the enzymatic extract of deer antler using Flavourzyme (2F) (100, 500 µg/mL), the IL-6 expression level was increased to 2.0 and 2.97 times, respectively, as compared to the normal control group (N). The increase at the concentration of 500 µg/mL was statistically significant (p < 0.01).

For the groups treated with the enzymatic extract of deer antler using Neutrase (3F) (500, 1000 µg/mL), the IL-6 expression level was increased significantly to 1.85 and 1.83 times, respectively, as compared to the normal control group (N) (p < 0.05 and p < 0.05).

For the groups treated with the enzymatic extract of deer antler using pepsin (4F) (100, 500, 2000 µg/mL), the groups treated with the enzymatic extract of deer antler using Protamex (5F) (100, 500, 2000 µg/mL) and the groups treated with the deer antler hot water extract (C2) (100, 500 µg/mL), there was no difference in the IL-6 expression level as compared to the normal control group (N) at all concentrations.

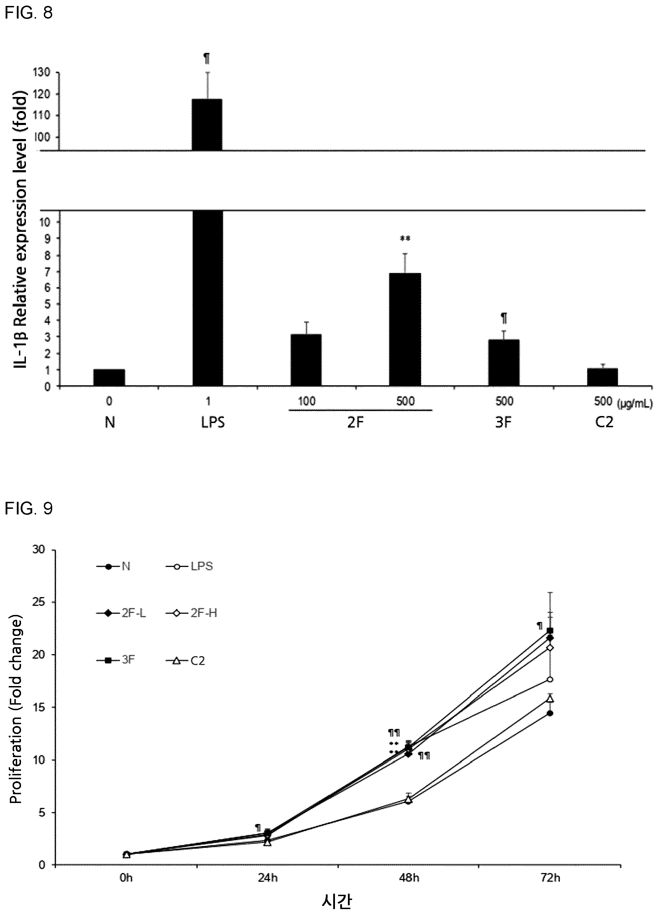

As seen from FIG. 8, the expression level of IL-1β in the positive control group (LPS 1 µg/mL) was increased statistically significantly as compared to the normal control group (N) (p < 0.05).

For the groups treated with the enzymatic extract of deer antler using Flavourzyme (2F) (100, 500 µg/mL), the IL-1β expression level was increased to 3.18 and 6.9 times, respectively, as compared to the normal control group (N). The increase at the concentration of 500 µg/mL was statistically significant (p < 0.01).

For the group treated with the enzymatic extract of deer antler using Neutrase (3F) (500 µg/mL), the IL-1β expression level was increased significantly to 2.82 times as compared to the normal control group (N) (p < 0.05), but the increase was lower by about 59.1% as compared to the group treated with the enzymatic extract of deer antler using Flavourzyme (2F) at the same concentration of 500 µg/mL.

For the group treated with deer antler hot water extract (C2) (500 µg/mL), there was no difference in the IL-1β expression level as compared to the normal control group (N) at all concentrations.

### Test Example 6: Ability of proliferating macrophages of enzymatic extracts of deer antler

Activated RAW 264.7 cells were seeded onto a 96-well plate at 5×10³ cells/well and cultured in a 5% CO₂ incubator at 37 °C for 24 hours. After replacing the medium with the positive control group or the enzymatic extract samples of deer antler prepared to different concentrations, the cells were cultured for 24 hours. Then, after adding 10 µL of CCK-8 (Cat No. CK04-13, Dojindo, Japan) and further culturing the cells in a 5% CO₂ incubator at 37 °C for 1 hour, cell proliferation was evaluated by measuring absorbance at 450 nm using Infinite 200 Pro (Tecan, Switzerland). The result is shown in FIG. 9.

The cell proliferation was measured at different times after the treatment with the test substances with respect to before the treatment (0 h), and statistical treatment was performed with respect to the normal control group at the corresponding times.

For the normal control group, the cells proliferated 2.35, 6.09 and 14.52-fold at 24, 48 and 72 hours, respectively. For the positive control group (LPS, 1 µg/mL), the cell proliferation increased to 2.8, 11.3 and 17.7 fold, respectively, at 24, 48 and 72 hours. Particularly, the cell proliferation increased statistically significantly at 24 hours and 48 hours as compared to the normal control group (p < 0.05 and p < 0.01).

For the groups treated with the enzymatic extract of deer antler using Flavourzyme (2F) (100, 500 µg/mL), the cell proliferation increased to 10.6 and 11.0 fold at 48 hours, respectively. At 48 hours, the cell proliferation was statistically significantly different from the normal control group (p < 0.01, p < 0.01). At 72 hours, the cell proliferation increased to 21.68 and 20.71 fold, respectively, as compared to the normal control group.

For the group treated with the enzymatic extract of deer antler using Neutrase (3F) (500 µg/mL), the cell proliferation increased statistically significantly to 11.2 and 22.4 fold at 48 and 72 hours, respectively, as compared to the normal control group (p < 0.01).

For the group treated with the deer antler hot water extract (C2) (500 µg/mL), no difference was observed from the normal control group at all times as 2.18, 6.34 and 15.90 fold.

### Test Example 7: Effect of administration of enzymatic extracts of deer antler to immunosuppression animal model on improvement of immunity

The effect of the enzymatic extracts of deer antler on the improvement of immunity in an immunosuppression animal model was investigated by measuring NK cell activity, interferon gamma (IFN-γ) concentration and TNF-α concentration.

Balb/c mice were divided into a normal control group (G1), a negative control group (G2) and test substance administration groups (G3-G9), with 6 mice per group, and the enzymatic extract of deer antler using Flavourzyme (2F) was administered at 50 mg/kg, 100 mg/kg or 200 mg/kg (G3, G4 and G5, respectively), the enzymatic extract of deer antler using Neutrase (3F) was administered at 100 mg/kg or 200 mg/kg (G6 and G7, respectively), or the deer antler hot water extract (C2) was administered at 100 mg/kg or 200 mg/kg (G8 and G9, respectively). The negative control group (G2) and the test substance administration groups (G3-G9) except for the normal control group were intraperitoneally administered with cyclophosphamide (CP) at 70 mg/kg for 3 days before the administration of the test substance and on day 13 after the administration of the test substance to induce immunosuppression. The test substance was orally administered for 14 days, once a day, and an excipient was orally administered instead of the test substance to the normal control group (G1) and the negative control group (G2). All the test groups were observed for general symptoms every day, and body weight was measured twice a week.

The body weight was decreased in the negative control group (G2) as compared to the normal control group (G1), whereas the test substance administration groups (G3-G9) showed no statistically significant difference in the body weight as compared to the negative control group (G2).

### 1. Enhancement of NK cell activity

On day 14 after the administration of the test substance, splenocytes were isolated from the spleen by conducting autopsy and NK cell activity was measured.

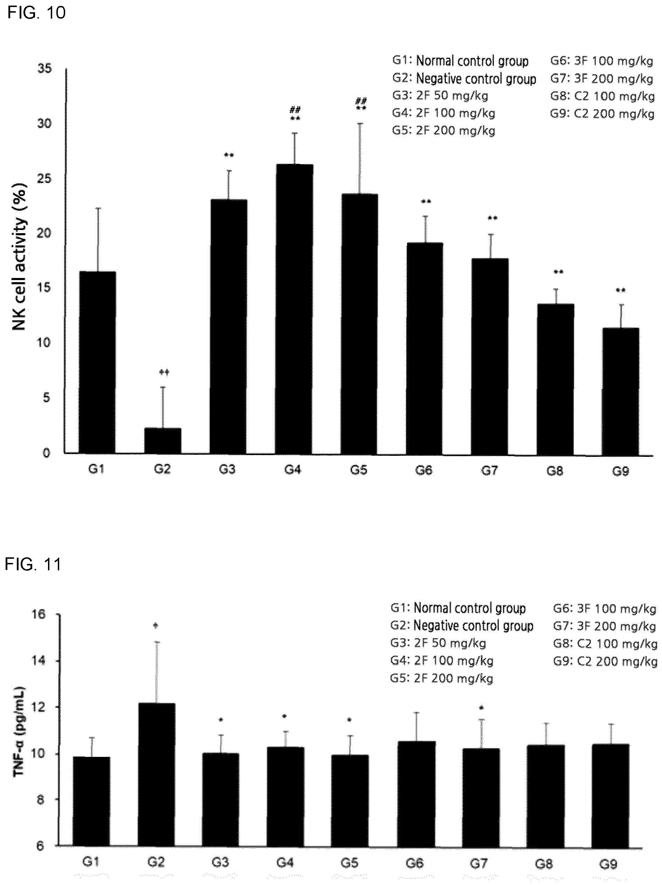

The NK cell activity was measured using a commercially available LDH assay kit (Cat. No.: 04744926001, Roche Diagnostics GmbH). The isolated splenocytes were used as effector cells, and YAC-1 mouse lymphoma cells were used as target cells. The effector cells (1×10⁶ cells/well) and the target cells (1×10⁴ cells/well) were mixed at a ratio of 50:1 and cultured for 4 hours. The amount of LDH released by the target cells is shown in FIG. 10.

It is known that NK cells play an important role in innate immunity. They directly kill the cells infected by microorganisms or cancer cells and activate monocytes and dendritic cells by secreting cytokines.

As seen from FIG. 10, whereas the NK cell activity of the normal control group (G1) was 16.5%, the NK cell activity of the negative control group (G2) was decreased statistically significantly to 2.3% as compared to the normal control group (p < 0.01).

For the groups G3, G4 and G5 to which the enzymatic extract of deer antler using Flavourzyme (2F) was administered at 50, 100 or 200 mg/kg, the NK cell activity was increased statistically significantly to 23.1, 26.4 and 23.7%, respectively, as compared to the negative control group (G2) (p < 0.01, p < 0.01 and p < 0.01). The increase of the NK cell activity was also statistically significant as compared to the deer antler hot water extract (G8, G9) of the same concentrations (p < 0.01 and p < 0.01).

For the groups G6 and G7 to which the enzymatic extract of deer antler using Neutrase (3F) was administered at 100 or 200 mg/kg, the NK cell activity was increased statistically significantly to 19.3 and 17.9%, respectively, as compared to the negative control group (G1) (p < 0.01 and p < 0.01). However, the NK cell activity was lower as compared the enzymatic extract of deer antler using Flavourzyme (2F) of the same concentrations and no statistically significant difference was observed as compared to the deer antler hot water extract (G8 and G9) of the same concentrations.

For the groups G8 and G9 to which the deer antler hot water extract (C2) was administered at 100 or 200 mg/kg, the NK cell activity was increased statistically significantly to 13.8 and 11.6%, respectively, as compared to the negative control group. However, the NK cell activity was lower as compared the enzymatic extract of deer antler using Flavourzyme (2F) and the enzymatic extract of deer antler using Neutrase (3F) of the same concentrations.

### 2. Analysis of immunocytokines in blood

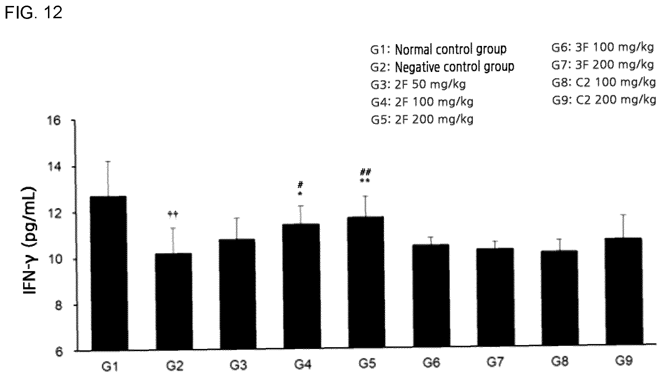

TNF-α and IFN-γ are representative cytokines secreted by Th1 cells. They are known to stimulate phagocytosis by increasing the activity of macrophages. The blood collected during the autopsy was kept at room temperature for 15-30 minutes and then centrifuged to separate serum. Then, the concentration of TNF-α and IFN-γ was measured using TNF-α (Cat. No.: MTA00B, R&D Systems) and IFN-γ (Cat. No.: MIF00, R&D Systems) ELISA kits according to the manufacturer's protocols. The result is shown in FIG. 11 and FIG. 12, respectively.

As seen from FIG. 11, the concentration of TNF-α in blood was 9.7 pg/mL for the normal control group (G1) and 12.2 pg/mL for the negative control group (G2). That is to say, the TNF-α concentration was increased statistically significantly in the immunosuppression-induced negative control group (G2) as compared to the normal control group (G1) (p < 0.01).

For the groups G3, G4 and G5 to which the enzymatic extract of deer antler using Flavourzyme (2F) was administered at 50, 100 or 200 mg/kg, the concentration of TNF-α was decreased significantly to 10.1, 10.3 and 10.0 pg/mL, respectively, as compared to the negative control group (G1) regardless of the concentration (p < 0.05, p < 0.05 and p < 0.05).

For the groups G6 and G7 to which the enzymatic extract of deer antler using Neutrase (3F) was administered at 100 or 200 mg/kg, the concentration of TNF-α was not significantly different from the negative control group (G2) as 10.6 and 10.3 pg/mL, at respectively, at the moderate concentration, but was decreased significantly as compared to the negative control group (G2) at the high concentration (p < 0.05).

Accordingly, it was confirmed that, whereas the concentration of TNF-α was decreased significantly in the group to which the enzymatic extract of deer antler using Flavourzyme (2F) was administered at the low concentration of 50 mg/kg as compared to the negative control group (G2), the TNF-α concentration was decreased significantly in the group to which the enzymatic extract of deer antler using Neutrase (3F) was administered at the high concentration of 200 mg/kg as compared to the negative control group (G2).

For the groups G8 and G9 to which the deer antler hot water extract (C2) was administered at 100 or 200 mg/kg, there was no difference in the TNF-α concentration from the negative control group (G1).

As seen from FIG. 12, the concentration of IFN-γ in blood was 12.7 pg/mL for the normal control group (G1) and 10.2 pg/mL for the negative control group (G2). That is to say, the concentration of IFN-γ was decreased statistically significant in the immunosuppression-induced negative control group (G2) as compared to the normal control group (G1) (p < 0.01).

For the groups G3, G4 and G5 to which the enzymatic extract of deer antler using Flavourzyme (2F) was administered at 50, 100 or 200 mg/kg, the IFN-γ concentration was increased to 10.8, 11.4 and 11.7 pg/mL, respectively, as compared to the negative control group (G2). In particular, the IFN-γ concentration was increased statistically significantly for G4 (100 mg/kg) and G5 (200 mg/kg) (p < 0.05 and p < 0.01). In addition, the IFN-γ concentration was also increased statistically significantly as compared to the deer antler hot water extract (G8 and G9) at the same concentration (p < 0.05 and p < 0.01).

For the groups G6 and G7 to which the enzymatic extract of deer antler using Neutrase (3F) was administered at 100 or 200 mg/kg and the groups G8 and G9 to which the deer antler hot water extract (C2) was administered at 100 or 200 mg/kg, there was no difference from the negative control group (G2).

Hereinafter, formulation examples of a composition containing the enzymatic extract of deer antler of the present disclosure are described. However, they are not intended to limit the present disclosure.

### Formulation Example 1: Preparation of powder

Powder of enzymatic extract of deer antler of Preparation Example 2 20 mg

| | |
|---|---|
| Lactose | 100 mg |
| Talc | 10 mg |

A powder was prepared by mixing the above ingredients and filling the mixture in a pouch.

### Formulation Example 2: Preparation of tablet

Powder of enzymatic extract of deer antler of Preparation Example 2 10 mg

| | |
|---|---|
| Cornstarch | 100 mg |
| Lactose | 100 mg |
| Magnesium stearate | 2 mg |

After mixing the above ingredients, a tablet was prepared according to a common tablet-making method.

### Formulation Example 3: Preparation of capsule

Powder of enzymatic extract of deer antler of Preparation Example 2 10 mg

| | |
|---|---|
| Crystalline cellulose | 3 mg |
| Lactose | 14.8 mg |
| Magnesium stearate | 0.2 mg |

After mixing the above ingredients, a capsule was prepared by filling the mixture in a gelatin capsule.

### Formulation Example 4: Preparation of granule

Powder of enzymatic extract of deer antler of Preparation Example 2 1,000 mg

| | |
|---|---|
| Vitamin mixture | Adequate |
| Vitamin A acetate | 70 µg |
| Vitamin E | 1.0 mg |
| Vitamin B₁ | 0.13 mg |
| Vitamin B₂ | 0.15 mg |
| Vitamin B₆ | 0.5 mg |
| Vitamin B₁₂ | 0.2 µg |
| Vitamin C | 10 mg |
| Biotin | 10 µg |
| Nicotinamide | 1.7 mg |
| Folic acid | 50 µg |
| Calcium pantothenate | 0.5 mg |
| Mineral mixture | Adequate |
| Ferrous sulfate | 1.75 mg |
| Zinc oxide | 0.82 mg |
| Magnesium carbonate | 25.3 mg |
| Monopotassium phosphate | 5 mg |
| Dicalcium phosphate | 55 mg |
| Potassium citrate | 90 mg |
| Calcium carbonate | 100 mg |
| Magnesium chloride | 24.8 mg |

The compositions of the vitamin and mineral mixtures are provided as preferred examples. However, they may be varied as desired. After mixing the above ingredients and preparing the mixture into a granule, a health functional food composition was prepared according to a common method.

### Formulation Example 5: Preparation of beverage

Powder of enzymatic extract of deer antler of Preparation Example 2 1,000 mg

| | |
|---|---|
| Citric acid | 1,000 mg |
| Oligosaccharide | 100 g |
| Plum concentrate | 2 g |
| Taurine | 1 g |
| Purified water | To 900 mL |

After mixing the above and heating the mixture at 85 °C for about 1 hour under stirring, the prepared solution was put in a sterilized 2-L container, sealed, sterilized and then stored in a refrigerator until use for preparation of a functional beverage composition according to the present disclosure.

## Claims

1. An immuno-potentiating food composition comprising an enzymatic extract of deer antler obtained by degrading deer antler with an enzyme of IUBMB EC 3.4.11.1 or an enzyme of IUBMB EC 3.4.24.28 as an active ingredient.

2. The immuno-potentiating food composition according to claim 1, wherein the enzyme is an enzyme of IUBMB EC 3.4.11.1.

3. The immuno-potentiating food composition according to claim 2, wherein the enzyme of IUBMB EC 3.4.11.1 is a complex of a protease and a peptidase derived from a mold.

4. The immuno-potentiating food composition according to claim 3, wherein the complex of a protease and a peptidase derived from a mold is a complex of an endoprotease and an exopeptidase derived from *Aspergillus oryzae.*

5. The immuno-potentiating food composition according to claim 4, wherein the complex of an endoprotease and an exopeptidase is Flavourzyme.

6. The immuno-potentiating food composition according to claim 1, wherein the enzyme of IUBMB EC 3.4.24.28 is a neutral protease derived from *Bacillus.*

7. The immuno-potentiating food composition according to claim 6, wherein the neutral protease derived from *Bacillus* is a neutral Zn metalloendoprotease produced by submerged fermentation of *Bacillus amyloliquefaciens.*

8. The immuno-potentiating food composition according to claim 7, wherein the neutral Zn metalloendoprotease is Neutrase.

9. The immuno-potentiating food composition according to any of claims 1 to 7, wherein the enzymatic extract of deer antler is an enzymatic extract of deer antler obtained by hydrolyzing dried antler obtained by freeze-drying raw deer antler or frozen deer antler by treating with an enzyme.

10. A method for preparing an enzymatic extract of deer antler having immuno-potentiating activity, comprising:
a freeze-dried deer antler preparation step of freeze-drying raw deer antler or frozen deer antler; and
an enzyme treatment step of adding 50-500 parts by weight of water and 0.01-1 part by weight of an enzyme to 10 parts by weight of the freeze-dried deer antler and conducting reaction at 30-60 °C for 1-48 hours,
wherein the enzyme is an enzyme of IUBMB EC 3.4.11.1 or an enzyme of IUBMB EC 3.4.24.28.

11. The method for preparing an enzymatic extract of deer antler having immuno-potentiating activity according to claim 10, wherein the enzyme is an enzyme of IUBMB EC 3.4.11.1.

12. An immuno-potentiating pharmaceutical composition comprising an enzymatic extract of deer antler obtained by degrading deer antler with an enzyme of IUBMB EC 3.4.11.1 or an enzyme of IUBMB EC 3.4.24.28 as an active ingredient.
